(19) Europäisches Patentamt
European Patent Office
Office européen des brevets

(11) **EP 2 441 492 A1**

(12) **EUROPEAN PATENT APPLICATION**

(43) Date of publication:
**18.04.2012 Bulletin 2012/16**

(51) Int Cl.:
***A61N 7/02*** *(2006.01)*

(21) Application number: **10187575.5**

(22) Date of filing: **14.10.2010**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Koninklijke Philips Electronics N.V. 5621 BA Eindhoven (NL)**

(72) Inventor: **Vuorinen, Reko 5600 AE, Eindhoven (NL)**

(74) Representative: **Damen, Daniel Martijn Philips Intellectual Property & Standards P.O. Box 220 5600 AE Eindhoven (NL)**

(54) **High intensity focused ultrasound system, computer-implemented method, and computer program product**

(57) A high intensity focused ultrasound system (300, 400) comprising: an ultrasound window (316, 416), an ultrasound transducer (306, 406) with multiple transducer elements (312) for focusing ultrasound energy into a focal volume (320, 420) within a subject, wherein the ultrasound transducer is adapted for directing ultrasound through the ultrasound window into the subject; an ultrasound transducer power supply (552) for adjusting the phase of electrical power supplied to each of the multiple transducer elements; a mechanical positioning system (310, 410) the ultrasound transducer; a processor (560); and a memory (564, 566) containing machine executable instructions (578, 580, 582, 584, 586). Execution of the instructions causes the processor to perform the steps of: moving (100, 208) the focal volume closer to the ultrasound transducer by controlling the ultrasound transducer power supply, and moving (102, 200) the ultrasound transducer closer to the ultrasound window by controlling the mechanical positioning system.

move the focal volume closer to the ultrasound transducer using the ultrasound power supply — 100

move the ultrasound transducer closer to the ultrasound window — 102

FIG. 1

EP 2 441 492 A1

# Description

## TECHNICAL FIELD

The invention relates to high intensity focused ultrasound, in particular a high intensity focused ultrasound system with reduced near field heating.

## BACKGROUND OF THE INVENTION

The selective heating of tissue in a subject may have therapeutic value. High intensity focused ultrasound therapy, which uses highly focused ultrasound has been successfully used to either heat or ablate tissue.

In high intensity focused ultrasound an array of transducer elements are used to form an ultrasonic transducer. Supplying alternating current electrical power to the transducer elements causes them to generate ultrasonic waves. The ultrasonic wave from each of the transducer elements either adds constructively or destructively. By controlling the phase of alternating current electrical power supplied to each of the transducer elements the focal point or volume into which the ultrasound power is focused may be controlled.

## SUMMARY OF THE INVENTION

The invention provides for a high intensity focused ultrasound system, a computer-implemented method, and a computer program product in the independent claims. Embodiments are given in the dependent claims.

A difficulty with using high intensity focused ultrasound (HIFU) is that areas which are not intended to be heated may be unintentionally heated. In particular the surface of a subject in contact with a high intensity focused ultrasound system may be heated or even burned by near field heating caused by the ultrasonic transducer. Embodiments of the invention address this problem and others by mechanically and electrically adjusting the focal volume of the ultrasonic transducer.

Embodiments may solve this problem and others by mechanically moving the ultrasonic transducer closer to an ultrasonic window of the high intensity focused ultrasound system and electronically moving the focal volume closer to the ultrasonic transducer to compensate for the mechanical translation of the ultrasonic transducer. This has the effect of spreading the ultrasonic energy or waves over a larger surface area in the near field region. Since the energy density is spread over a larger area there is less chance that any particular region will be inadvertently heated.

One of the main problems in HIFU is the near field heating i.e., part of the energy is absorbed in the tissue between the transducer and the focus. This absorption is caused by several factors

    1. Normal absorption of ultrasound in a tissue.

    2. Acoustical reflections at surfaces with different acoustical impedance (skin-fat-muscle interfaces) increase acoustical intensity at the interface.

    3. Diffusion of heat from the focal region towards the transducer.

Embodiments of the invention may address issues 1 and 2 above.

As energy absorption is directly proportional to beam intensity (this assumption is valid at low intensities, as in near field), a straightforward way to improve the situation is to maximize the beam cross section. This can be accomplished by keeping the transducer as close to the patient skin as possible, while using electronic deflection to maintain the heating at the desired location.

In principle one could also use some other means to deflect the focus e.g., mechanical deformations on the transducer face/assembly, to achieve the same effect

Normally the transducer position is determined by its geometry i.e., the focal length, and the chosen angle of incidence. In the simplest case, where sonication is performed at direct angle with respect to skin, the beam cross section can be approximated by a simple geometrical calculation

$$A(x) = A_0 \cdot (x/f)^2$$

Here $A_0$ = transducer active area ($A_0 = \pi D^2/4$, where $D$ is transducer aperture), $A(x)$ = beam cross section at distance $x$ from the focus (in the near field, towards the transducer), and $f$ = focal distance (distance from focus to the transducer aperture).

In practice the focal distance is determined by the deepest penetration required for the given application. However, it is typical that most of the sonications are performed to much smaller depth (closer to the skin) than this. By using negative electronic deflection, it is possible to sonicate nearby structures while keeping the transducer closer to the patient skin. For deflection $\delta$, we can calculate the near field beam cross section $A_d(x, \delta)$ as:

$$A_d(x, \delta) = A_0 \cdot [(x + \delta)/f]^2$$

Comparing the beam cross sections in deflected and non-deflected case, we get:

$$A_d(x, \delta)/A(x) = [(x + \delta)/x]^2$$

Using numbers e.g., $\delta$ = 1 cm, $x$ = 4 cm, we get $A_d(4, 2)/A(4)$ = 1.56. As heating is inversely proportional to beam cross section, this example would yield a 36% reduction for the energy deposited at distance of 4 cm from the focus.

A computer-readable storage medium as used herein is any storage medium which may store instructions which are executable by a processor of a computing device. The computer-readable storage medium may be a computer-readable non-transitory storage medium. The computer-readable storage medium may also be a tangible computer readable medium. A computer-readable storage medium may also be referred to as 'memory.' In some embodiments, a computer-readable storage medium may also be able to store data which is able to be accessed by the processor of the computing device. An example of a computer-readable storage medium include, but are not limited to: a floppy disk, a magnetic hard disk drive, a solid state hard disk, flash memory, a USB thumb drive, Random Access Memory (RAM) memory, Read Only Memory (ROM) memory, an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network.

Computer memory is an example of a computer-readable storage medium. Computer memory is any memory which is directly accessible to a processor. Examples of computer memory include, but are not limited to: RAM memory, registers, and register files.

Computer storage is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa.

A 'processor' as used herein encompasses an electronic component which is able to execute a program or machine executable instruction. References to the computing device comprising "a processor" should be interpreted as possibly containing more than one processor. The term computing device should also be interpreted to possibly refer to a collection or network of computing devices each comprising a processor. Many programs have their instructions performed by multiple processors that may be within the same computing device or which may even distributed across multiple computing device.

A medical imaging device as used herein encompasses a device or apparatus for acquiring medical imaging data. Medical imaging data as used herein encompasses data, images, or information which is descriptive of the internal structure and/or is descriptive of the anatomical structure of a subject.

Magnetic resonance data is defined herein as being the recorded measurements of radio frequency signals emitted by atomic spins by the antenna of a Magnetic resonance apparatus during a magnetic resonance imaging scan. Magnetic resonance data is an example of medical imaging data. A Magnetic Resonance Imaging (MRI) image is defined herein as being the reconstructed two or three dimensional visualization of anatomic data contained within the magnetic resonance imaging data. This visualization can be performed using a computer.

In one aspect the invention provides for a high intensity focused ultrasound system. The high intensity focused ultrasound system comprises an ultrasound window. As used herein an ultrasound window is a window which is able to transmit ultrasonic waves or energy. Typically a thin film or membrane is used as an ultrasound window. The ultrasound window may for example be made of a thin membrane of BoPET (Biaxially-oriented polyethylene terephthalate).

The high intensity focused ultrasound system further comprises an ultrasound transducer. The ultrasound transducer has multiple transducer elements for focusing ultrasound energy into a focal volume. Each of the multiple transducer elements may be independently supplied with electrical energy. When the transducer elements are supplied with electrical energy they produce ultrasound radiation. The multiple transducer elements are arranged such that the ultrasound energy is focused into a focal volume.

The high intensity focused ultrasound system further comprises an ultrasound transducer power supply for supplying electrical power to each of the multiple transducer elements. The ultrasound transducer power supply is adapted for adjusting the phase of electrical power supplied to each of the multiple transducer elements. By controlling the phase of electrical power supplied to each of the multiple transducer elements the location of the focal volume may be adjusted. This is because the ultrasound energy is essentially ultrasonic waves which are generated by the multiple transducer elements. If the phase of the individual multiple transducer elements are adjusted then the ultrasound energy coming from each of the transducer elements may add constructively or destructively depending upon the phase.

Some embodiments of the high intensity focused ultrasound system may further comprise a fluid filled volume in contact with the multiple transducer elements and the ultrasound window. The ultrasound transducer may be adapted for directing ultrasound energy through the ultrasound window into the subject. The focal volume may be within the subject. Another way of wording this is that the focal volume lies beyond the ultrasound window. The fluid filled volume serves to conduct the ultrasound energy or waves to the ultrasound window. As used herein an ultrasound window is a window which is able to transmit ultrasonic waves or energy. Typically a thin film or membrane is used as an ultrasound window. The ultrasound window may for example be made of a thin membrane of BoPET (Biaxially-oriented polyethylene terephthalate).

The high intensity focused ultrasound system further comprises a mechanical positioning system for mechanically positioning the ultrasound transducer relative to the ultrasound window. In some embodiments the mechanical positioning system may be manually actuated. In other embodiments the mechanical positioning system may be actuated by an automated system. The mechanical positioning system may comprise a system for actuating and mechanically adjusting the position of the ultrasound transducer. The high intensity focused ultrasound system further comprises a processor for controlling the high intensity focused ultrasound system. The high intensity focused ultrasound system further comprises a memory containing machine executable instructions for execution by the processor. Execution of the instructions causes the processor to perform the step of moving the focal volume closer to the ultrasound transducer by controlling the ultrasound transducer power supply. The ultrasound transducer power supply is adapted such that the phase of the electrical power supplied to each of the transducer elements may be adjusted. The focal volume is moved closer to the ultrasound transducer by adjusting the phase. Execution of the instructions further causes the processor to perform the step of moving the ultrasound transducer closer to the ultrasound window by controlling the mechanical positioning system. This may be accomplished for instance by the instructions having a command sequence or code which is sent by the processor to the mechanical positioning system.

This embodiment is particularly advantageous because the focal volume has been moved closer to the ultrasound transducer and the ultrasound transducer has been moved closer to the ultrasound window. This means that the distance between the ultrasound transducer and focal volume has been reduced. This may have the effect of reducing the near field heating by the high intensity focused ultrasound system. For instance if the target were within the subject and there was a fat layer or skin intermediate to the focal volume then performing the steps of the invention may reduce the near field heating. This is because the energy flux due to the ultrasonic energy is spread over larger surface areas of the subject. This has reduced the effect of ultrasonic energy being concentrated in regions where it is undesired or where it is not desired to heat tissue using the ultrasound transducer.

In another embodiment the focal volume is moved closer to the ultrasound transducer and the ultrasound transducer is moved closer to the ultrasound window for the reduction of near field heating by the ultrasound transducer.

In another embodiment the focal volume is moved closer to the ultrasound transducer by controlling the phase of the electrical power supply to each of the multiple transducer elements. As was mentioned before, the ultrasound energy is in the form of ultrasonic waves. By changing the phase the waves may add or cancel each other destructively according to the phase at different locations. Controlling the phase allows the focal volume to be moved or focused in different regions without physically moving the ultrasound transducer.

In another embodiment execution of the instructions further causes the processor to perform the step of calculating a set of phases of electrical power to be supplied to each of the multiple transducer elements by using ray tracing. The step of moving the focal volume closer to the ultrasound transducer is performed by controlling the ultrasound transducer power supply in accordance with the set of phases. Ray tracing as used herein encompasses calculating the intensity of ultrasonic energy within the location of a subject or in a path to the subject taking into account the propagation of the ultrasound and its phase. In other words ray tracing encompasses taking into account the phase of the ultrasound energy from the different transducer elements of the ultrasound transducer.

In another embodiment the high intensity focused ultrasound system further comprises a medical imaging system for acquiring medical imaging data. Execution of the instructions further causes the processor to perform the step of acquiring medical imaging data using the medical imaging system. The ray tracing is performed in accordance with the medical image data. It is advantageous to use the medical image data for the ray tracing because different regions of the subject may have different ultrasound properties. For instance the tissue properties of fat are different from that of muscle.

In another embodiment the medical imaging system is a magnetic resonance imaging system.

In another embodiment the medical imaging system is a computed tomography system. The computed tomography system may be an X-ray computed tomography system.

In another embodiment the medical imaging system is an ultrasound imaging system.

In some embodiments the same ultrasound transducer may be used for imaging as for generating the ultrasonic energy which is focused into the focal volume. In other embodiments there is a separate ultrasound transducer for generating the ultrasonic energy and a separate ultrasound transducer for performing the acquisition of medical imaging data.

In another embodiment the medical imaging system is a positron emission tomography system.

In another embodiment the medical imaging system is a combined magnetic resonance imaging system and/or computed tomography system and/or ultrasound imaging system and/or positron emission tomography system.

In another embodiment execution of the instructions further causes the processor to perform the step of constructing an ultrasound model using the medical image data. An ultrasound model as used herein encompasses a model which models the ultrasonic properties of a subject. For instance, an ultrasonic model may model the reflection of ultrasound at boundaries between different regions of a subject. An ultrasonic model may also model the transmission and/or attenuation of ultrasound by different regions of a subject. An ultrasonic model may model the local absorption of ultrasonic energy by a subject. Described in other terms, an ultrasonic model is a model which may be used to mathematically predict the transmission, attenuation, energy absoption, and/or reflection of ultrasound in a subj ect.

The ray tracing is performed using the ultrasound model. As was mentioned before the use of the medical image data to construct the ultrasound model is advantageous because the medical imaging data may reveal or detail the anatomical structure of the subject.

In another embodiment the high intensity focused ultrasound system further comprises a fluid filled volume in contact with the multiple transducer elements and the ultrasound window. Execution of the instructions further causes the processor to perform the step of constructing an ultrasound model using knowledge of the geometry of the ultrasound transducer, the fluid filled volume, and the ultrasound window. The ray tracing is performed using the ultrasound model. This embodiment is particularly advantageous when the geometry of the ultrasound transducer and the fluid filled volume is a known quantity. This embodiment may significantly improve the quality of the sonication of the focal volume without the use of expensive medical imaging.

In another embodiment the execution of the instructions further cause the processor to perform the step of modeling an ultrasound response at an ultrasound impedance interface using the ultrasound model. Execution of the instructions further cause the processor to perform the step of modifying the set of phases calculated during the ray tracing in accordance with the ultrasound response at the ultrasound impedance interface. An ultrasound impedance interface as used herein encompasses a boundary between two different regions which have a different response to ultrasound energy. For instance muscle or fat tissue adjacent to bone would be an example of an ultrasound impedance interface. Another example of an ultrasound impedance interface is the boundary between the fluid filled volume and the ultrasound window. The phases calculated during the ray tracing take into account the ultrasound response at the ultrasound impedance interface which in other words takes into account the reflection of ultrasound energy by the ultrasound impedance interface. This is advantageous because whenever the impedance or the response to ultrasonic energy changes there may be a reflection of ultrasound energy or waves.

In another embodiment execution of the instructions further comprise performing a sonication of the focal volume using the ultrasound transducer. This is an advantageous embodiment because the focal volume is heated using ultrasound energy. This may be used for a variety of therapies or for warming the focal volume. In another embodiment there is a target volume. The target volume may be larger than the focal volume. Execution of the instructions further cause the processor to perform the step of performing a volumetric sonication of the target volume by dynamically controlling the phase of electrical power supplied to the multiple transducer elements. In some embodiments the mechanical positioning system may also be used to move the ultrasound transducer during the volumetric sonication of the target volume.

In another aspect the invention provides for a computer-implemented method of controlling a high intensity focused ultrasound system according to an embodiment of the invention. The method comprises electronically moving the focal volume closer to the ultrasound transducer by controlling the ultrasound transducer power supply. The method further comprises moving the ultrasound transducer closer to the ultrasound window by controlling the mechanical positioning system.

In another aspect the invention provides for a method of controlling a high intensity focused ultrasound system according to an embodiment of the invention. The method comprises electronically moving the focal volume closer to the ultrasound transducer by controlling the ultrasound transducer power supply. The method further comprises moving the ultrasound transducer closer to the ultrasound window by controlling the mechanical positioning system.

In another aspect the invention provides for a computer program product comprising machine executable instructions for execution by the processor of a high intensity focused ultrasound system according to an embodiment of the invention. The computer program product may be stored for instance on a computer-readable storage medium. Execution of the instructions causes the processor to perform the step of electronically moving the focal volume closer to the ultrasound transducer by controlling the ultrasound transducer power supply. Execution of the instructions further causes the processor to perform the step of moving the ultrasound transducer closer to the ultrasound window by controlling the mechanical positioning system.

BRIEF DESCRIPTION OF THE DRAWINGS

In the following preferred embodiments of the invention will be described, by way of example only, and with reference to the drawings in which:

Fig.1 shows a block diagram which illustrates an embodiment of the method according to the invention;
Fig. 2 shows a block diagram which illustrates a further embodiment of the method according to the invention;
Figs. 3a and 3b show cross-sectional views of a high intensity focused ultrasound system according to an embodiment of the invention,

Fig. 4 shows a cross-sectional view of a high intensity focused ultrasound system according to a further embodiment of the invention; and

Fig. 5 shows a functional diagram of a high intensity focused ultrasound system that is integrated with a magnetic resonance imaging system according to an embodiment of the invention.

## DETAILED DESCRIPTION OF THE EMBODIMENTS

Like numbered elements in these figures are either equivalent elements or perform the same function. Elements which have been discussed previously will not necessarily be discussed in later figures if the function is equivalent.

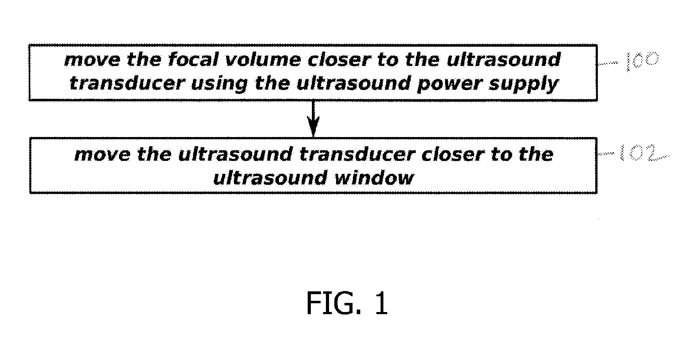

Fig. 1 shows a block diagram which illustrates an embodiment of the method according to the invention. In step 100 the focal volume is moved closer to the ultrasound transducer using the ultrasound power supply. This may be accomplished in some embodiments by adjusting the phase of individual transducer elements of the ultrasound transducer. In step 102 the ultrasound transducer is moved closer to the ultrasound window using a mechanical positioning system.

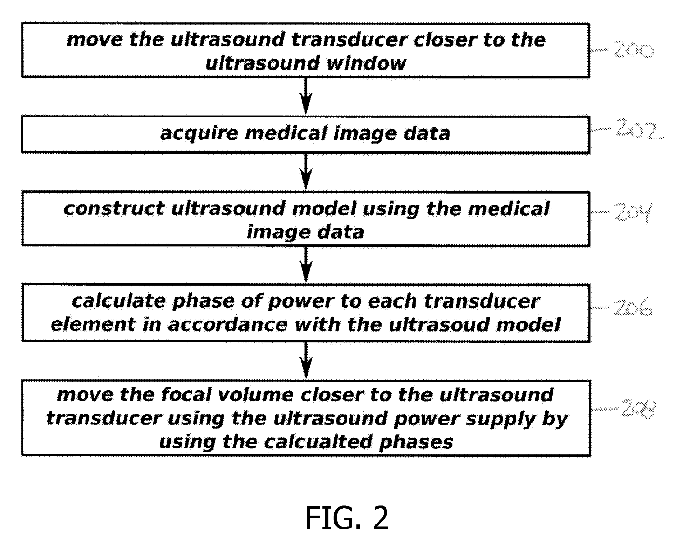

Fig. 2 shows a block diagram which illustrates a further embodiment of a method according to the invention. In step 200 the ultrasound transducer is moved closer to the ultrasound window using a mechanical positioning system. In step 202 medical image data is acquired. In step 204 an ultrasound model is constructed using the medical image data. In step 206 the phase of power at each transducer is calculated in accordance with the ultrasound model. The phases are calculated such that the focal volume will move closer to the ultrasound transducer. Finally in step 208 the focal volume is moved closer to the ultrasound transducer using the ultrasound power supply by using the calculated phases. That is to say the ultrasound power supply controls the phases of the individual transducer elements of the ultrasound transducer such that the focal volume moves closer to the ultrasound transducer.

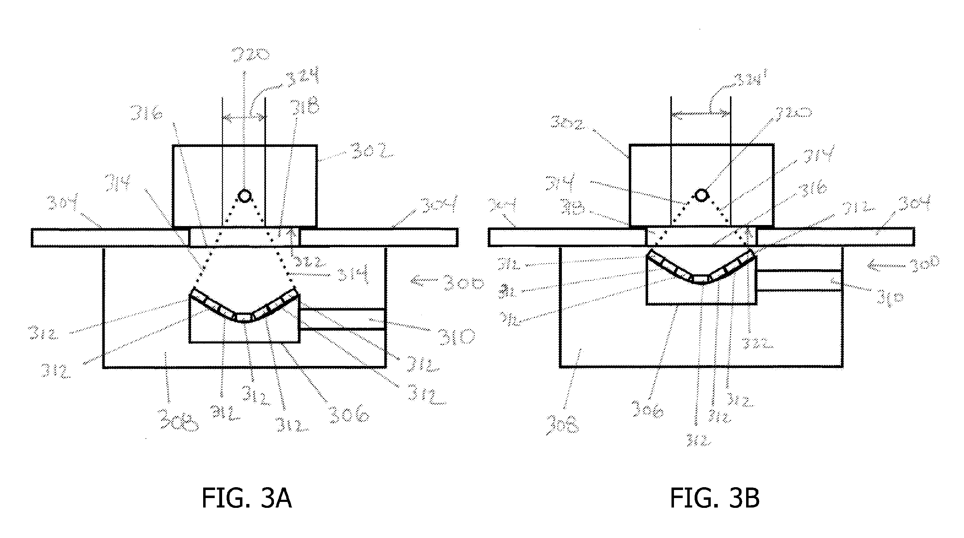

Figs. 3a and 3b show cross-sectional views of a high intensity focused ultrasound system 300 according to an embodiment of the invention. Figs. 3a and b illustrate how an embodiment of the invention may reduce near field heating in a subject 302.

In both Figs. 3a and 3b there is a high intensity focused ultrasound system 300. There is a subject 302 reposing on a subject support 304. The high intensity focused ultrasound system 300 is mounted below the subject support 304. There is an ultrasound transducer 306 mounted within a fluid filled volume 308 of the high intensity focused ultrasound system 300. There is a mechanical positioning system 310 for moving the ultrasound transducer 306 relative to an ultrasound window 316. The ultrasound transducer 306 has multiple transducer elements 312 which are used for generating ultrasound energy. The dashed lines marked 314 show the path of ultrasound energy from the ultrasound transducer 306. The ultrasound energy passes through the fluid filled volume 308 and then through an ultrasound window 316. In this embodiment the ultrasound window 316 is shown as being in contact with a gel pad 318. A gel pad is a pad which is designed for conducting ultrasound energy between an ultrasound window 316 and a subject 302. In other embodiments the subject 302 may be in direct contact with the ultrasound window 316. In other embodiments the subject 302 may be in contact with the ultrasound window 316 through ultrasound conductive gel or other fluid. The ultrasound energy is shown as being focused into a focal volume 320.

In both Figs. there is a surface of the subject 322 in contact with the gel pad 318. The arrow 324 illustrates the size of the cross-section through which the ultrasound energy passes. In Fig. 3b the mechanical positioning system 310 has been used to move the ultrasound transducer 306 closer to the ultrasound window 316 than in Fig. a. However, the phase of electrical energy supplied to the transducer elements in Fig. 3b has been adjusted such that the focal volume 320 is identical in both Figs. 3a and 3b. The cross-section of the surface of the subject 324 in Fig. 3b is larger than the cross-section 324 in Fig. 3a. The same amount of ultrasound energy in both examples is flowing to the focal volume 320. It can be seen that the same amount of energy is flowing through a larger surface area in Fig. 3b than in Fig. 3a. The effect of this is that the near heating, particularly at the surface of the subject 322, is reduced in Fig. 3b. This illustrates how an embodiment of the invention may reduce the near field heating and also in particular reduce the likelihood that the subject 302 will receive a burn at the surface of the subject 322.

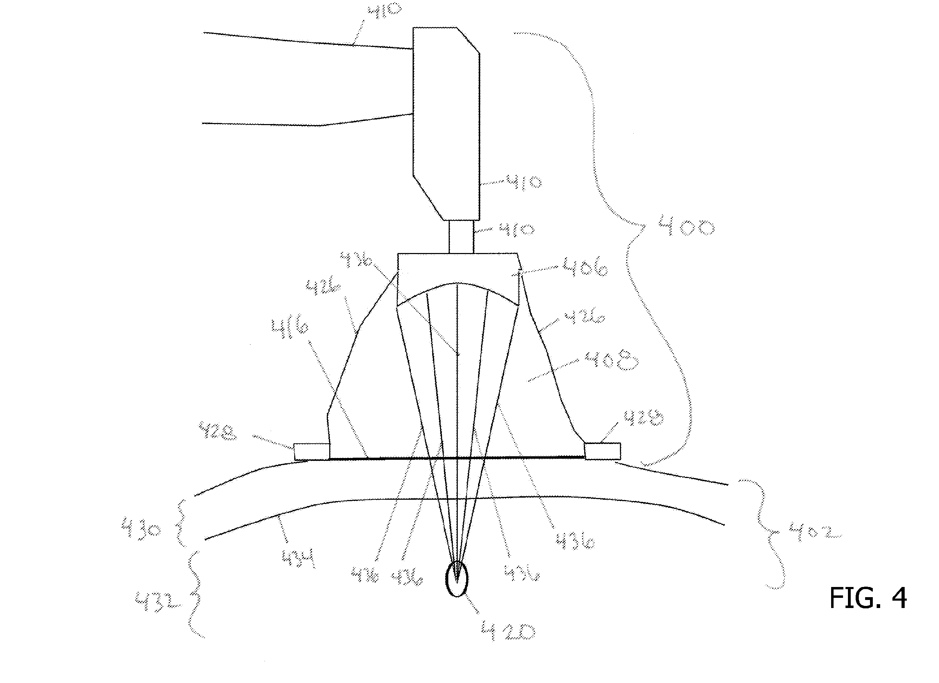

Fig. 4 shows a functional diagram of a high intensity focused ultrasound system 400 according to a further embodiment of the invention. In this embodiment there is an ultrasound transducer 406 immersed in a fluid filled volume 408. The ultrasound transducer 406 is positioned using a robotic positioning system 410. The robotic positioning system 410 moves the ultrasound transducer 406 relative to an ultrasound window 416. In this embodiment the fluid filled volume 408 is surrounded by a membrane 426. The ultrasound window 416 is connected to a support ring 428 as is the membrane 426. The ultrasound window 416 is in contact with a subject 402. In this embodiment the subject 402 has several different regions.

The ultrasound window 416 is in contact with a first layer of the subject 430. The first layer of the subject is in contact with a second layer of the subject 432. The first layer 430 and the second layer 432 comprise different materials or tissue types so there is an ultrasound impedance interface 434 between the two of them. For calculation of the phases for transducer elements in this figure. Rays 436, which trace the path from the ultrasound transducer 406 to a focal volume 420, are shown. To calculate the phase accurately in the focal volume 420 transmission of ultrasound through the first layer 430 and the second layer 432 both need to be considered. By accurately calculating the phase in the focal volume 420 for each of the transducer elements the velocity of the ultrasound in the first layer 430, the second layer 432 and the fluid filled volume 408 are all considered.

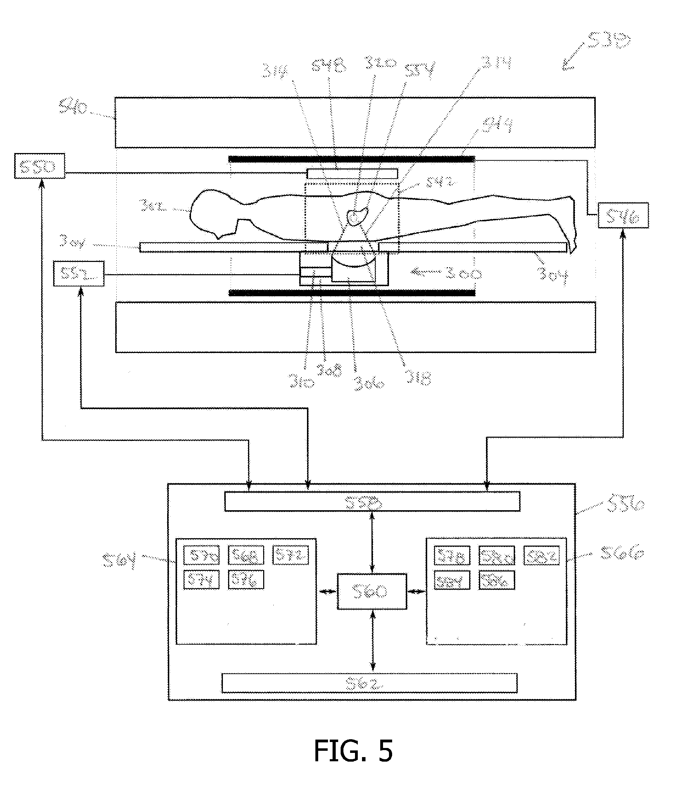

Fig. 5 shows a functional diagram of a high intensity focused ultrasound system 300 that is integrated with a magnetic resonance imaging system 538 according to an embodiment of the invention. In this example a magnetic resonance imaging system 538 is used. However other medical imaging systems may also be integrated into the high intensity focused ultrasound system 300. For instance the magnetic resonance imaging system may be replaced by a computed tomography system, an ultrasound imaging system, a positron emission tomography system, or a combined magnetic resonance imaging system and/or computed tomography system and/or ultrasound imaging system and/or positron emission tomography system.

The high intensity focused ultrasound system 300 is equivalent shown to those in Figs. 3a and 3b. Not all of the details shown in Figs. 3a and 3b are repeated in this Fig.

There is a subject 302 reposing on a subject support 304 and the subject support 304 is above the high intensity focused ultrasound system 300. Both the subject and the high intensity focused ultrasound system 300 are within the bore of a magnet 540 of the magnetic resonance imaging system 538. The magnetic resonance imaging system further comprises a magnetic field gradient coil 544. The magnetic field gradient coil in this Fig. represents three separate coil systems, the x, y and z gradient coils. The magnetic field gradient coil 544 is shown as being connected to a magnetic field gradient coil power supply 546. The magnetic field gradient coil power supply 546 supplies current to the three coil systems which make up the magnetic field gradient coil 544. The magnet 540 has an imaging zone 542 where the magnetic field of the magnet 540 is uniform enough that magnetic resonance imaging may be performed.

Adjacent to the imaging zone 542 is a radio frequency coil 548. The radio frequency coil 548 may represent a single coil or may also represent individual transmit and receive coils. The radio frequency coil 548 is shown as being connected to a radio frequency transceiver 550. Likewise the radio frequency transceiver 550 represents possibly independent transmitter and an independent receiver. The high intensity focused ultrasound system 300 is shown as being connected to an ultrasound transducer power supply 552. The ultrasound transducer power supply 552 supplies power to each element of the ultrasound transducer 306. The ultrasound transducer power supply 552 may be able to control the phase of electrical power supplied to each of the transducer elements of the ultrasound transducer 306. The ultrasound transducer power supply 552 may also be able to supply control signals to the mechanical positioning system 310.

The magnetic field gradient coil power supply 546, the radio frequency transceiver 550 and the ultrasound transducer power supply 552 are shown as being connected to a hardware interface 558 of a computer system 556. The computer system 556 comprises a processor connected to the hardware interface 558. The processor 560 is able to send and receive signals to the high intensity focused ultrasound system 300 and the magnetic resonance imaging system 538 via the hardware interface 558. Essentially the processor 560 is able to control the high intensity focused ultrasound system 300 and the magnetic resonance imaging system 538 using the hardware interface 558. The computer system 556 further comprises a user interface 562. The user interface comprises hardware which allows a user or operator to interact with and control the computer system 556. The computer system 556 further comprises computer storage 564 and computer memory 566 which are both accessible by the processor 560.

The computer storage 564 is shown as containing magnetic resonance data 568 which is acquired using the magnetic resonance imaging system 538. The computer storage 564 is further shown as containing a treatment plan 570. The treatment plan 570 contains instructions or details created by a physician or care giver which may be used to create a set of control sequences 576 for performing the volumetric sonication of the target volume 554. The computer storage 564 is further shown as containing an ultrasound model 574. The ultrasound model 574 is constructed by segmenting the magnetic resonance image 572. The computer storage 564 is further shown as containing a control sequence 576. The control sequence 576 contains machine executable instructions which the processor 560 may send to the high intensity focused ultrasound system 300 and/or magnetic resonance imaging system 538 during sonication of the target volume 554. The control sequence may comprise a set of phases for setting the phase of alternating electrical power delivered to the transducer elements during sonication. In some embodiments the system is able to modify the control sequence 576 during the sonication of the target volume 554.

The computer memory 566 is shown as containing a magnetic resonance control module 578. The magnetic resonance control module 578 contains machine executable instructions usable by the processor 560 for generating control sequences for controlling the operation of the magnetic resonance imaging system 538. The computer memory 566 is further shown as containing an ultrasound control module 580. The ultrasound control module 580 contains machine executable instructions for use by the processor 560 for generating control commands for operating the high intensity focused ultrasound system 300. The computer memory 566 is further shown as containing a magnetic resonance image reconstruction module 582. The magnetic resonance image reconstruction module 582 contains machine executable instructions for reconstructing the magnetic resonance data 568 into a magnetic resonance image 572. The computer memory 566 is further shown as containing an ultrasound model creation module 584. The ultrasound model creation module 584 contains machine executable instructions which enable to segment the magnetic resonance image 572 and create the ultrasound module 574. Finally the computer memory 566 is shown as containing a control sequence creation module 586. The control sequence creation module 586 is able to use the treatment plan 570 and the ultrasound model 574 for generating the control sequence 576.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

300    high intensity focused ultrasound system

302    subject

304    subject support

306    ultrasound transducer

308    fluid filled volume

310    mechanical positioning system

312    transducer element

314    path of ultrasound energy

316    ultrasound window

318    gel pad

320    focal volume

322    surface of subject

324    cross section of surface of subject

400    high intensity focused ultrasound system

402    subject

406    ultrasound transducer

408    fluid filed volume

410    robotic positioning system

416    ultrasound window

420    focal volume

426    membrane

428    support ring

430    first layer of subject

432    second layer of subject

434    ultrasound impedance interface

436    ray

538    magnetic resonance imaging system

540    magnet

542    imaging zone

544    magnetic field gradient coil

546    magnetic field gradient coil power supply

548    radio frequency coil

550    radio frequency transceiver

552    ultrasound transducer power supply

554    target volume

556    computer system

558    hardware interface

560 processor

562 user interface

564 storage 566 memory

568 magnetic resonance data

570 treatment plan

572 magnetic resonance image

574 ultrasound model

576 control sequence

578 magnetic resonance control module

580 ultrasound control module

582 magnetic resonance image reconstruction module

584 ultrasound model creation module

586 control sequence creation module

**Claims**

1. A high intensity focused ultrasound system (300, 400) comprising:

   - an ultrasound window (316, 416);
   - an ultrasound transducer (306, 406) with multiple transducer elements (312) for focusing ultrasound energy into a focal volume (320, 420) within a subject (302, 402), wherein the ultrasound transducer is adapted for directing ultrasound through the ultrasound window into the subject;
   - an ultrasound transducer power supply (552) for supplying electrical power to each of the multiple transducer elements, wherein the ultrasound transducer power supply is adapted for adjusting the phase of electrical power supplied to each of the multiple transducer elements;
   - a mechanical positioning system (310, 410) for mechanically positioning the ultrasound transducer relative to the ultrasound window;
   - a processor (560) for controlling the high intensity focused ultrasound system; and
   - a memory (564, 566) containing machine executable instructions (578, 580, 582, 584, 586) for execution by the processor, wherein execution of the instructions causes the processor to:
   - move (100, 208) the focal volume closer to the ultrasound transducer by controlling the ultrasound transducer power supply, and
- move (102, 200) the ultrasound transducer closer to the ultrasound window by controlling the mechanical positioning system.

2. The high intensity focused ultrasound system of claim 1, wherein the focal volume is moved closer to the ultrasound transducer and the ultrasound transducer is moved closer to the ultrasound window such that near field heating by the ultrasound transducer is reduced.

3. The high intensity focused ultrasound system of claim 1, 2, or 3, wherein the focal volume is moved closer to the ultrasound transducer by controlling the phase of the electrical power supplied to each of the multiple transducer elements.

4. The high intensity focused ultrasound system of claim 3, wherein execution of the instructions further causes the processor to calculate (206) a set of phases (576) of electrical power to be supplied to each of the multiple transducer elements by using ray tracing, wherein moving the focal volume closer to the ultrasound transducer is performed by controlling the ultrasound transducer power supply in accordance with the set of phases.

5. The high intensity focused ultrasound system of claim 4, wherein the high intensity focused ultrasound system further comprises a medical imaging system for acquiring medical imaging data (568), wherein the instructions further cause the processor to acquire (202) medical image data using the medical imaging system, and wherein the ray tracing is performed in accordance with the medical image data.

6. The high intensity focused ultrasound system of claim 5, wherein the medical imaging system is any one of the following: a magnetic resonance imaging system, an computed tomography system, an ultrasound imaging system, a positron emission tomography system, and a combined magnetic resonance imaging system and/or computed tomography system and/or ultrasound imaging system, and/or positron emission tomography system.

7. The high intensity focused ultrasound system of claim 5 or 6, wherein execution of the instructions further causes the processor to construct (204) an ultrasound model (574) using the medical image data, and wherein the ray tracing is performed using the ultrasound model.

8. The high intensity focused ultrasound system of any one of claims 1 through 4; wherein the high intensity focused ultrasound system further comprises a fluid filled volume (308, 408) in contact with the multiple transducer elements and the ultrasound window; wherein execution of the instructions further causes the processor to construct an ultrasound model (574) using a knowledge of the geometry of the ultrasound transducer, the fluid filled volume, and the ultrasound window; and wherein the ray tracing is performed using the ultrasound model.

9. The high intensity focused ultrasound system of claim 5, 6, 7, or 8, wherein execution of the instructions further cause the processor to:

- model an ultrasound response at an ultrasound impedance interface (434) using the ultrasound model, and
- modify the set of phases calculated during the ray tracing in accordance with the ultrasound response at the ultrasound impedance interface.

10. The high intensity focused ultrasound system of any one of the preceding claims, wherein execution of the instructions further cause the processor to perform a sonication of the focal volume by controlling the ultrasound transducer.

11. The high intensity focused ultrasound system of claim 10, wherein there is a target volume (554), wherein execution of the instructions further cause the processor to perform a volumetric sonication of the target volume by dynamically controlling the phase of electrical power supplied to the multiple transducer elements.

12. A computer-implemented method of controlling a high intensity focused ultrasound system according to any one of claims 1 through 11, wherein the method comprises:

- moving (100, 208) the focal volume closer to the ultrasound transducer by controlling the ultrasound transducer power supply, and
- moving (102, 200) the ultrasound transducer closer to the ultrasound window by controlling the mechanical positioning system.

13. A computer program product comprising machine executable instructions for execution by a processor of a high intensity focused ultrasound system according to any one of claims 1 through 11, wherein execution of the instructions causes the processor to:

- move (100, 208) the focal volume closer to the ultrasound transducer by controlling the ultrasound transducer power supply, and
- move (102, 200) the ultrasound transducer closer to the ultrasound window by controlling the mechanical positioning system.

move the focal volume closer to the ultrasound transducer using the ultrasound power supply — 100

move the ultrasound transducer closer to the ultrasound window — 102

FIG. 1

```
┌─────────────────────────────────────────────────┐
│   move the ultrasound transducer closer to the   │ ─── 200
│              ultrasound window                    │
└─────────────────────────────────────────────────┘
                         │
                         ▼
┌─────────────────────────────────────────────────┐
│            acquire medical image data             │ ─── 202
└─────────────────────────────────────────────────┘
                         │
                         ▼
┌─────────────────────────────────────────────────┐
│    construct ultrasound model using the medical   │ ─── 204
│                   image data                      │
└─────────────────────────────────────────────────┘
                         │
                         ▼
┌─────────────────────────────────────────────────┐
│    calculate phase of power to each transducer    │ ─── 206
│   element in accordance with the ultrasoud model  │
└─────────────────────────────────────────────────┘
                         │
                         ▼
┌─────────────────────────────────────────────────┐
│    move the focal volume closer to the ultrasound │ ─── 208
│   transducer using the ultrasound power supply by │
│          using the calcualted phases              │
└─────────────────────────────────────────────────┘
```

# FIG. 2

FIG. 3A

FIG. 3B

FIG. 4

FIG. 5

Europäisches
Patentamt

European
Patent Office

Office européen
des brevets

## EUROPEAN SEARCH REPORT

**Application Number**

EP 10 18 7575

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/103472 A1 (INSIGHTEC IMAGE GUIDED TREAT L [IL]; VORTMAN KOBI [IL]; VITEK SHUKI [I) 2 December 2004 (2004-12-02)<br>* abstract; figure 2 *<br>* page 6, line 19 - page 23, line 5 *<br>----- | 1-11,13 | INV.<br>A61N7/02 |
| X | WO 01/80709 A2 (TXSONICS LTD [IL])<br>1 November 2001 (2001-11-01)<br>* abstract *<br>* page 5, line 2 - line 12 *<br>* page 6, line 26 - page 12, line 15 *<br>----- | 1-11,13 | |
| A | US 2008/200806 A1 (LIU HAO-LI [TW] ET AL)<br>21 August 2008 (2008-08-21)<br>* abstract *<br>* paragraph [0063] - paragraph [0072] *<br>----- | 4-11 | |
| A | WO 2010/029479 A1 (KONINKL PHILIPS ELECTRONICS NV [NL]; KOEHLER MAX O [FI]; VAARA TEUVO J) 18 March 2010 (2010-03-18)<br>* abstract *<br>* page 3, line 20 - line 32 *<br>----- | 4-11 | TECHNICAL FIELDS SEARCHED (IPC)<br>A61N |
| A | US 2009/171252 A1 (BOCKENSTEDT CRAIG ROBERT [US] ET AL)<br>2 July 2009 (2009-07-02)<br>* abstract *<br>* paragraph [0056] - paragraph [0058] *<br>----- | 1-11 | |
| A | WO 2006/110388 A1 (BOSTON SCIENT SCIMED INC [US]; OSTROVSKY ISAAC [US]; FAIRNENY TY [US];) 19 October 2006 (2006-10-19)<br>* abstract *<br>* paragraph [0016] - paragraph [0018] *<br>* paragraph [0038] *<br>----- | 1-11 | |

The present search report has been drawn up for all claims

| Place of search | Date of completion of the search | Examiner |
|---|---|---|
| Munich | 24 March 2011 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 18 7575

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2011

| Patent document cited in search report | | | Publication date | Patent family member(s) | | | Publication date |
|---|---|---|---|---|---|---|---|
| WO 2004103472 | A1 | | 02-12-2004 | AT<br>EP<br>US<br>US | 485875<br>1624934<br>2004236253<br>2010056962 | T<br>A1<br>A1<br>A1 | 15-11-2010<br>15-02-2006<br>25-11-2004<br>04-03-2010 |
| WO 0180709 | A2 | | 01-11-2001 | AT<br>AU<br>CN<br>EP<br>JP<br>US | 397958<br>5061801<br>1444491<br>1274483<br>2004507280<br>6613004 | T<br>A<br>A<br>A2<br>T<br>B1 | 15-07-2008<br>07-11-2001<br>24-09-2003<br>15-01-2003<br>11-03-2004<br>02-09-2003 |
| US 2008200806 | A1 | | 21-08-2008 | NONE | | | |
| WO 2010029479 | A1 | | 18-03-2010 | NONE | | | |
| US 2009171252 | A1 | | 02-07-2009 | NONE | | | |
| WO 2006110388 | A1 | | 19-10-2006 | EP<br>JP<br>US | 1866032<br>2008535568<br>2006241530 | A1<br>T<br>A1 | 19-12-2007<br>04-09-2008<br>26-10-2006 |

EPO FORM P0459

For more details about this annex : see Official Journal of the European Patent Office, No. 12/82